# EUROPÄISCHE PATENTANMELDUNG

(11) **EP 1 099 413 A1**
(43) Veröffentlichungstag der Anmeldung: **16.05.2001**
(21) Anmeldenummer: 99122289.4
(22) Anmeldetag: 09.11.1999
(51) Int. Cl.: A61B 17/17

(54) **Distales Zielgerät-System für die Verriegelung von intramedullären Knochennägeln**

(71) Anmelder: Schlumbohm Medizin-Labor-Technologie GmbH, 21079 Hamburg (DE)
(72) Erfinder: Schlumbohm, Hans-Joachim, 21266 Festeburg (DE)
(74) Vertreter: Fleck, Thomas, Dr. Dipl.-Chem.

(57) **Zusammenfassung**

Die Erfindung betrifft ein distales Zielgerät-System (100) für die Verriegelung von intramedullären Knochennägeln am Femur und/oder Tibia sowie Humerus und/oder Radius, mit einem Verriegelungsnagelsystem. Im Stand der Technik sind derartige Systeme in unterschiedlichen Ausführungsformen vorbekannt und weisen im Prinzip den Nachteil auf, daß Fehlbohrungen auftreten können, durch die die Patienten unnötig leiden müssen. Auch sind die auf dem Markt befindlichen Verriegelungsnagelsysteme nicht für jedes Zielgerät verwendbar. Bei diesen Problemen des Standes der Technik schafft die Erfindung Abhilfe dadurch, daß ein Zuggerät (50-56) mit Spindel (54) und Extensions(hand)schuh (50), sowie röntgenstrahlendurchlässige Unterlagen (22+30) für den Femur und/oder Tibia bzw. den Arm vorgesehen werden, wobei die Femurunterlage (30) direkt und das Zugaggregat (56) und die Tibia- oder Armunterlage (22) über ein Stativ (40-44) am Operationstisch (65) im Gebrauchszustand befestigt sind, und daß ein zur Positionierung in den Strahlengang eines Röntgendurchleuchtungsgerätes bringbares Zielgerät (1-17,90) mit einer auf einem Aufnahmezapfen (13) angeordneten Bohrschablone (14-17), mittels eines Steuerkopfes (2-12) fixiert wird, der Regel- und Arretierungsschrauben (8+12) zur horizontalen und vertikalen Verschiebung der Bohrschablone (14-17) aufweist, am Operationstisch (65) im Gebrauchszustand mittels Befestigungseinrichtungen (1) befestigt ist, wobei mehrere Reduktionshülsen (18) mit unterschiedlichem Durchmesser, sowie Arbeitshülse (77) mit Zentrierhülse (76) und Trokar (75) vorgesehen werden, deren Durchmesser auf die Bohrungen (15) in der Bohrschablone (14-16) zur zielgerichteten Positionierung in Richtung des Nagelloches abgestimmt sind.

## Beschreibung

Die vorliegende Erfindung betrifft ein distales Zielgerät-System für die Verriegelung von intramedullären Knochennägeln am Femur und/oder Tibia, sowie Humerus und/oder Radius, mit einem Verriegelungsnagelsystem.

Derartige distale Zielgerät-Systeme sind in den unterschiedlichsten Ausführungsformen im Stand der Technik bekannt. Im folgenden sollen einige vorbekannte Systeme und ihre Nachteile beschrieben werden.

Die DE-U-296 18 227 betrifft eine Zielvorrichtung für die Verschraubung von Verriegelungsnägeln am Femur und Tibia, die aus Haltezangen besteht, welche im proximalen und distalen Teil der zu versorgenden Extremitäten angebracht und zusätzlich mit einem parallel laufenden Stangensystem verbunden werden, welches parallel zu den Implantaten liegen muß. Die Zielvorrichtung muß so fixiert werden, daß sich die in einer Metallscheibe befindlichen Aussparungen über die Schraubenöffnungen des implantierten Nagels unter Durchleutung darstellen lassen. Erst dann kann die zur Fixierung benötigte Schraube eingebracht werden. Diese Zielvorrichtung beinhaltet einen hohen technischen Aufwand, der trotzdem zu Fehlbohrungen führen kann, da Relativbewegungen zwischen den Extremitäten und der Zielvorrichtung nicht ausgeschlossen werden können.

Auch die DE-U-296 08 071 beschreibt ein Zielgerät zum Positionieren und Einbringen von osteosynthetischen Befestigungselementen, insbesondere von Verriegelungsschrauben oder Bolzen in der Fusspartie eines in einen Knochen eingebrachten Marknagels mit einem zum lösbaren Verbinden mit dem Kopfende des Marknagels bestimmten Zielbügel und mit einer, mit Hilfe eines Kupplungsmittels daran verbundenen longitudinalen Zielschiene mit mindestens einer Führungsbohrung und eine am zweiten Abschnitt der Zielschiene fixierbare erste Abstandsgebereinheit mit einem Abstandsgeber, dessen Auflagefläche auf die Oberfläche des Marknagels in dessen Fußpartie eine longitudinale Verschiebung quer zum Marknagel zuläßt. Die Anbringung dieses Zielsystems ist ebenfalls sehr aufwendig und kann Fehlbohrungen nicht verhindern. Ein weiterer Nachteil dieses Zielgerätes liegt darin, daß es ausschließlich für einen bestimmten Nagel mit bestimmten Abmessungen geeignet ist.

Die DE-A1-42 385 82 beschreibt ein distales Zielgerät bei Verriegelungsnagelungen in der Chirurgie zum Einsetzen der quer durch das Nagelende verlaufenden Bolzen mit einem Führungsteil für die Bohrer, das an einem an dem Knochen angebrachten Extensionsbügel höhen- und seitenbeweglich und verschwenkbar befestigt ist. Unvorteilhaft ist hierbei, daß der schon vorgeschädigte Knochen durch eine weitere Bohrung, die nur zur Befestigung dient, zusätzlich in Mitleidenschaft gezogen wird.

Die DE-U-84 17 428 betrifft ein Zielgerät zur Herstellung von Querbohrungen im Knochen in Übereinstimmung mit Löchern oder Bohrungen eines Osteosynthesehilfsmittels im Knochen, insbesondere eines Verriegelungsnagels, mit einer Halterung zur Aufnahme eines in den Strahlengang eines Röntgengerätes bringbaren Zielglieds, wobei an der Halterung ein Aufnahmekopf aus einem für Röntgenstrahlung durchscheinenden Werkstoff drehbar gelagert ist zur Aufnahme eines Bohrers oder eines Bohrdrahtes und an der Halterung ferner eine kraftgetriebene Antriebsmaschine zum drehenden Antrieb des Aufnahmekopfes angeordnet ist. Der Nachteil dieses Gerätes und seiner Verfahrensweise liegt darin, daß es sich um eine Freihandhaltung handelt, die immer wieder zu Fehlbohrungen führt.

Das überwiegende Problem des Standes der Technik stellen also die immer wieder auftretenden Fehlbohrungen dar, die es gilt auszuschließen. Ein weiteres Ziel der Erfindung liegt darin, eine einfache, unkomplizierte und schnelle Montage des eingangs genannten distalen Zielgerät-Systems zu ermöglichen, das für jedes auf dem Markt befindliche Verriegelungsnagel-System (mit Schrauben oder Bolzen) geeignet ist, das eine Bohrung im distalen Teil von etwa 1,5 bis 6 mm Durchmesser aufweist. Das genannte Problem bzw. die Aufgabe der Erfindung und Ihre Zielstellung wird bei dem eingangs genannten distalen Zielgerät-System dadurch gelöst, daß ein Zuggerät mit Spindel und Extensions(hand)schuh, sowie röntgenstrahlendurchlässige Unterlagen für den Femur und/oder Tibia bzw. den Arm vorgesehen werden, wobei die Femurunterlage direkt und das Zugaggregat und die Tibia- oder Armunterlage über ein Stativ am Operationstisch im Gebrauchszustand befestigt sind, und daß ein zur Positionierung in den Strahlengang eines Röntgendurchleuchtungsgerätes bringbares Zielgerät mit einer auf einem Aufnahmezapfen angeordneten Bohrschablone, mittels eines Steuerkopfes fixiert wird, der Regel- und Arretierungsschrauben zur horizontalen und vertikalen Verschiebung der Bohrschablone aufweist, am Operationstisch im Gebrauchszustand mittels Befestigungseinrichtungen befestigt ist, wobei mehrere Reduktionshülsen mit unterschiedlichem Durchmesser, sowie Arbeitshülse mit Zentrierhülse und Trokar vorgesehen werden, deren Durchmesser auf die Bohrungen in der Bohrschablone zur zielgerichteten Positionierung in Richtung des Nagelloches abgestimmt sind. Von entscheidender erfindungsgemäßer Bedeutung ist danach die Merkmalskombination der Befestigung bestimmter Teile im Gebrauchszustand am Operationstisch selbst, die dafür verantwortlich sind, die zu operierende Extremität fest auf dem Opterationstisch zu fixieren, um Relativbewegungen jeglicher Art zwischen dem Zielgerät-System und der zu operierenden Extremität oder durch auf den OP-Tisch einwirkende Stösse mit Sicherheit auszuschließen. Fehlbohrungen können somit erfindungsgemäß vollständig ausgeschlossen werden. Auch bedarf das erfindungsgemäße Zielgerät-System darüber hinaus keiner zusätzlichen Fixierung am Knochen. Das System eignet sich auch für anterior posterior verlaufende Verriegelungen.

Mit dem erfindungsgemäßen Zielgerät-System können selbstverständlich auch proximale Verriegelungen ohne weiteres Zubehör vorgenommen werden. Allerdings ist der Arbeitsbereich vorherrschend ein schwieriger distaler Bereich.

Ebenfalls ist es in erfinderischer Hinsicht wichtig, daß das Zielgerät in Übereinstimmung der distalen Löcher des Verriegelungsnagelsystems unter Durchleutung angebracht und eingestellt wird.

Weitere Vorteile und Merkmale gehen aus den Unteransprüchen hervor, die auch gemeinsam mit dem Hauptanspruch von erfinderischer Bedeutung sein können.

Im folgenden wird ein bevorzugtes Ausführungsbeispiel anhand der Zeichnung näher erläutert, die dem besseren Verständnis der Erfindung dienen sollen, auf das die Erfindung per se jedoch nicht beschränkt ist. Es zeigt:
- Fig. 1: eine Schemaskizze des erfindungsgemäßen Zielgerät-Systems mit Steuerkopf und Bohrschablone, wie es an der Unterschenkel- oder Oberschenkelbeinauflage befestigt ist;
- Fig. 2: eine Querschnittsansicht und eine Draufsicht auf eine Reduktionshülse;
- Fig. 3: eine Querschnittsansicht und eine entsprechende Draufsicht auf die Unterlage für den Unterschenkel mit Befestigungseinrichtung für das Zugaggregat sowie das Zielgerät;
- Fig. 4: eine Draufsicht und eine Querschnittsansicht durch die Unterlage für den Oberschenkel;
- Fig. 5: eine Querschnittsansicht durch das Teleskopstativ;
- Fig. 6: eine Querschnittsansicht des Zugspindelaggregates mit dem Schnürschuh ohne Spitze mit verkürzter Schaftlänge;
- Fig. 7: eine Seitenansicht auf die vertikale Stange;
- Fig. 8: eine schematische Seitenansicht des erfindungsgemäßen Zielgerät-Systems wie es kurz vor Gebrauch am OP-Tisch befestigt ist;
- Fig. 9: eine schematische Ausschnittsansicht des Nagelsystems;
- Fig. 10 bis 12: schematische Ausschnittsansichten von Bohrschablone und Nagelloch;
- Fig. 13 bis 15: schematische Ausschnittsansichten des Nagels mit seinen Löchern in der Draufsicht und Verfahrensablauf mit Trokar;
- Fig. 16: eine Ausschnittsansicht von Bohrschablone mit Nagel und Trokar;
- Fig. 17: eine Ausschnittsansicht von Nagel und Trokar mit Arbeits- und Zentrierhülse;
- Fig. 18: eine Ausschnittsansicht der Bohrschablone mit Arbeitshülse;
- Fig. 19: eine Ausschnittsansicht von Nagel und eingesetzter Arbeitshülse; und
- Fig. 20: eine Ausschnittsansicht von eingesetztem Nagel mit durch die Arbeitshülse eingesetzter Schraube und Schraubendreher.

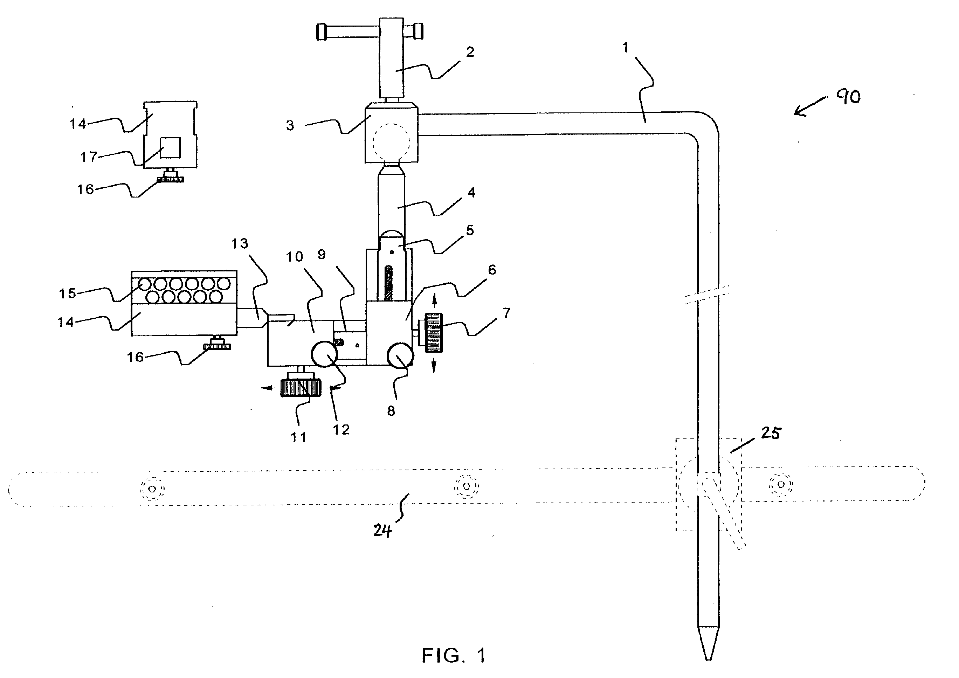

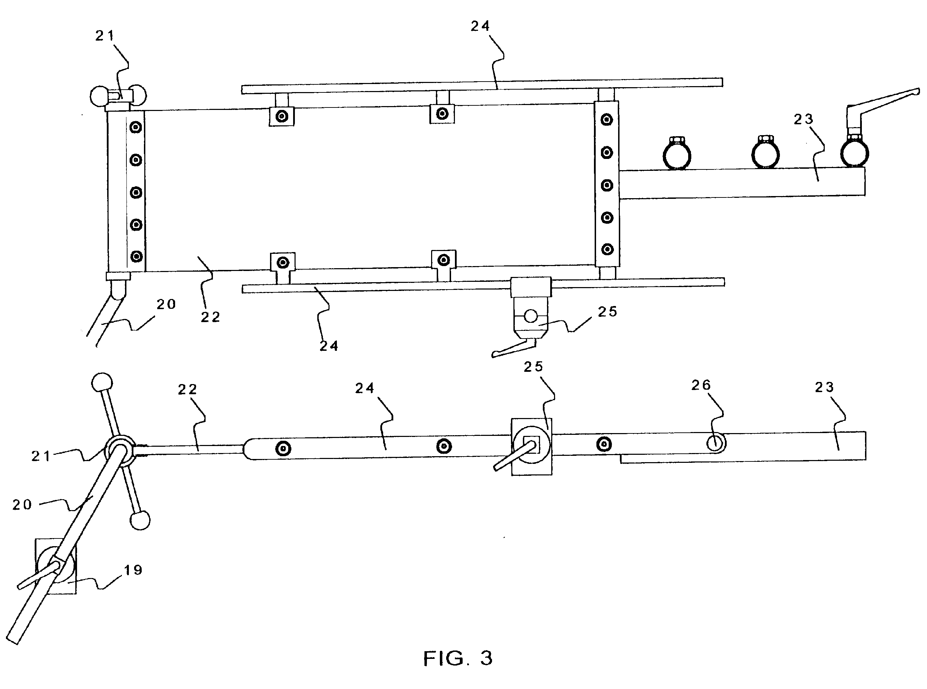

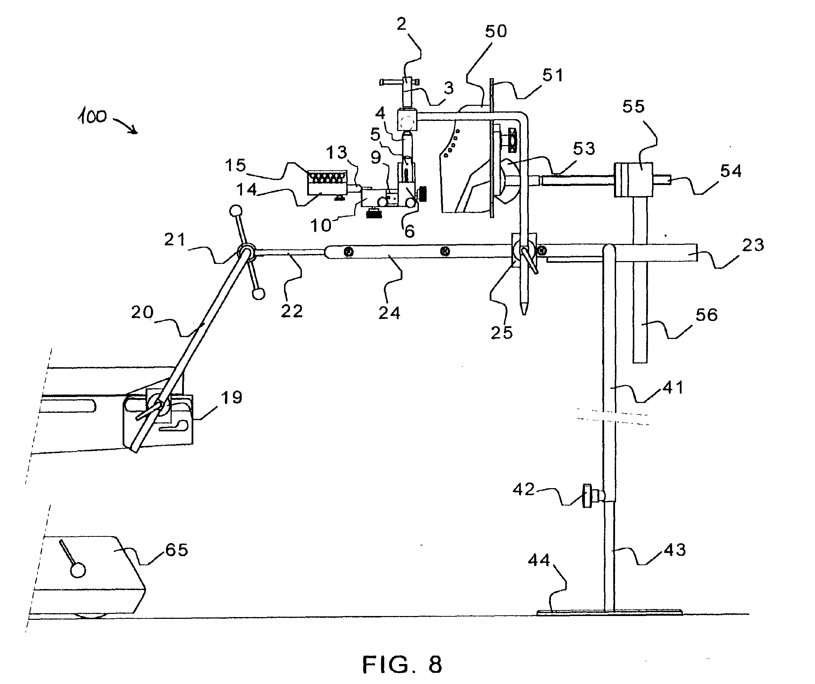

In Fig. 1 ist ein Teil des erfindungsgemäßen distalen Zielgerät-Systems, nämlich das in den Strahlengang eines Röntgendurchleuchtungsgerätes bringbare Zielgerät, allgemein mit 90 bezeichnet. Das vollständige Zielgerät-System 100 gibt die Fig. 8 wieder. Das Zielgerät 90 besteht aus den Merkmalen 1 bis 17, nämlich einer Befestigungsstange 1, die über eine autoklavierbare Radialstellklemme 25 mit einer seitlichen Geräteschiene 24 der in Fig. 3 gezeigten Unterschenkelbeinauflage im Bereich des einen Endes und mit ihrem anderen Ende mit einem quaderartigen Druckknopf 3 verbunden ist. Eine Knebelschraube 2 dient zum Arretieren der gestrichelt dargestellten Gelenkkugel. Am unteren Ende weist die Gelenkkugel eine Anschlußstange 4 auf, die mit einer Grundplatte 5 für die vertikale Verstellung mit eingelegter Zahnstange und Schwalbenschwanzführung verbunden ist. Unterhalb der Grundplatte 5 liegt ein vertikaler Verstellblock 6 mit Schwalbenschwanzaufnahme und einer seitlich angeordneten Rendelschraube zur vertikalen Verstellung, wie es durch die Pfeile angedeutet ist. Mit der Arretierschraube 8 kann die vertikale Verstellung blockiert werden. Eine Grundplatte 9 dient zur horizontalen Verstellung und weist ebenfalls eine eingelegte Zahnstange und eine Schwalbenschwanzführung auf. Der daran seitlich angrenzende horizontale Verstellblock 10 weist eine Schwalbenschwanzaufnahme auf und eine Rendelschraube 11 dient zur horizontalen Verstellung. Mit der Arretierschraube 12 kann die horizontale Verstellung blockiert werden. Ein Aufnahmequader oder Aufnahmezapfen 13 wird für die Bohrschablone 14 vorgesehen, die als Bohrschablonenblock ausgebildet ist und elf Bohrlöcher 15 mit einem Durchmesser von 9,5 mm aufweist. Mit einer Arretierschraube 16 kann die Bohrschablone auf dem Aufnahmezapfen 13 arretiert werden. Über der Bohrschablone 14 ist dieselbe um 90° gedreht abgebildet, so daß man die quadratische Durchdringung bzw. Bohrung 17 zur Einführung des Aufnahmezapfens 13 erkennt.

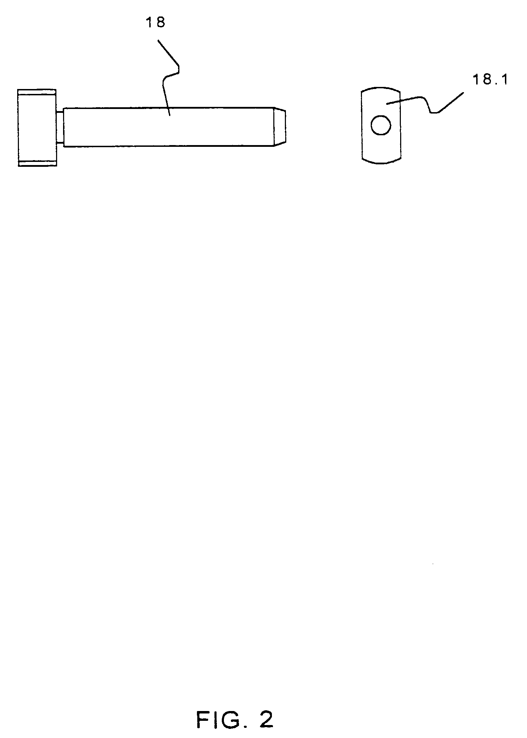

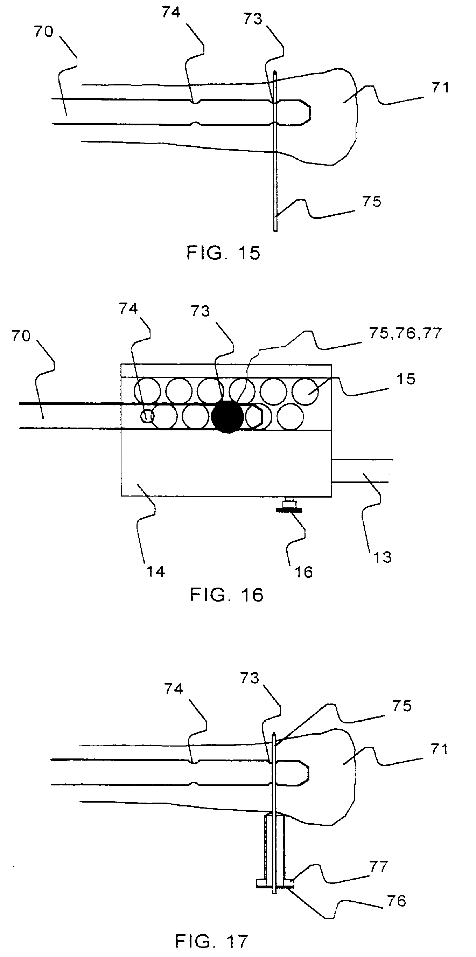

Kernstück des erfindungsgemäßen Zielgerätes 90 ist also der Steuerkopf 2 bis 12 mit der Bohrschablone 14. In die für die jeweilige Operation erforderliche Richtung und Stellung wird die Bohrschablone 14 auf den quadratischen Aufnahmezapfen 13 des Steuerkopfes geschoben und fixiert. Die Bohrungen 15 der Schablone 14 sind auf die identischen Durchmesser der in Fig. 2 gezeigten Reduktionshülsen 18, der Arbeitshülse 77 (vgl. Fig. 16 und 17) und des Skalpellhalters (nicht gezeigt) exakt abgestimmt. Es sind vier Reduktionshülsen 18 mit einem Außendurchmesser von 9,533 mm und einem Innendurchmesser 18.1 von 2,7 mm, 3,5 mm, 4,0 mm und 5 mm vorgesehen. Der Innendurchmesser der Zentrierhülse 76 (vgl. Fig. 16 und 17) nimmt einen Trokar 75 auf, wobei die Zentrierhülse 76 exakt in die Arbeitshülse 77 paßt. Es bleibt bei der Einführung der vorgenannten Teile in die Bohrschablone 14 nur so wenig Spiel, daß eine genaue zielgerichtete Positionierung in Richtung des Knochens gewährleistet ist. Die grobe Positionierung der Bohrschablone 14 erfolgt über den Druckknopf 3 mit Spannring zum Arretieren der Gelenkkugel (gestrichelt dargestellt), während die Feinabstimmung in vertikaler und horizontaler Richtung mittels in Schwalbenschwanzführung liegenden Zahnstangen über Rendelschrauben 7 und 12 vorgenommen wird.

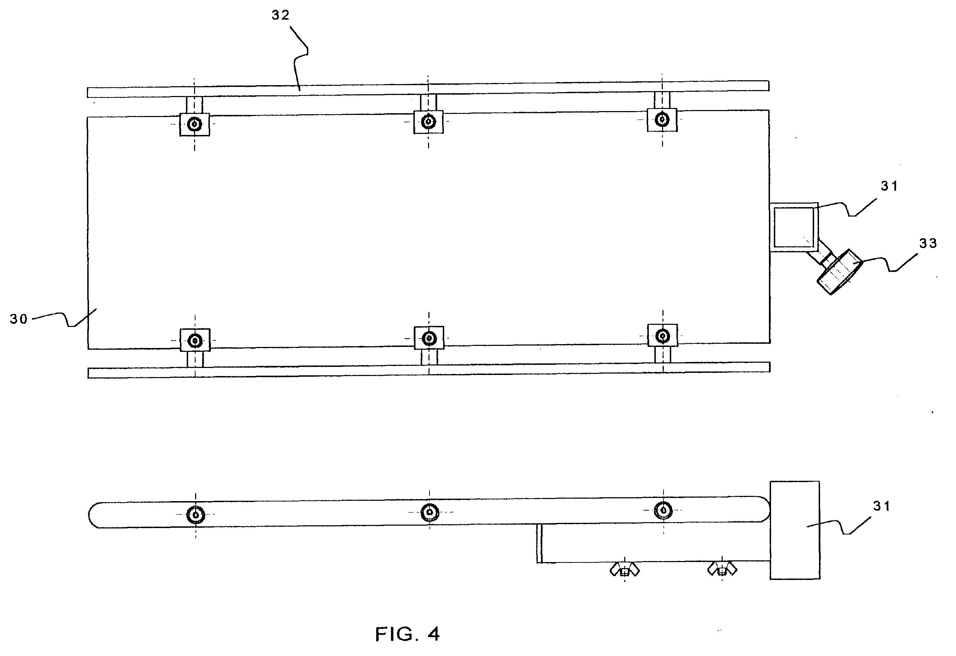

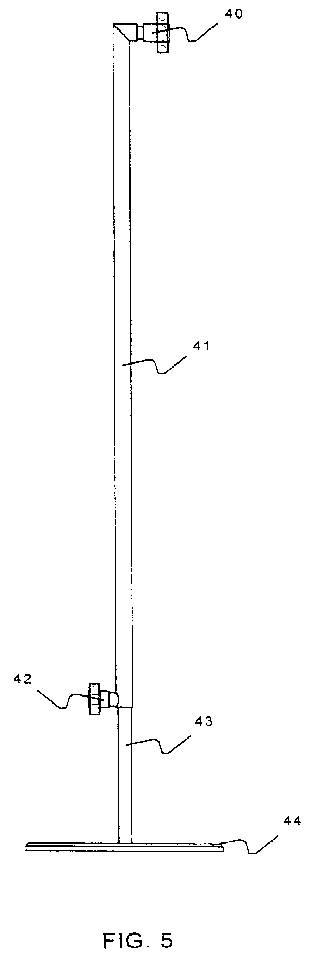

Von besonderer erfindungsgemäßer Bedeutung ist es, daß das Zielgerät 90 mit seinen Zubehöhrteilen am Operationstisch 65 im Gebrauchszustand mittels der Befestigungsstange 1 befestigt ist. Ebenso ist es wichtig, daß die zu operierende Extremität (nicht gezeigt) für die Dauer der Operation fest fixiert wird, nämlich durch eine Unterschenkel- bzw. Oberschenkelunterlage 22 bzw. 30 (vgl. Fig. 3 bzw. 4). Ferner geht die Erfindung davon aus, daß die Positionierung unter Röntgendurchleuchtung vorgenommen wird. Fig. 3 zeigt in zwei Ansichten die erfindungsgemäß eingesetzte Unterschenkelbeinauflage, die auch als Beinplattenunterlage 22 bezeichnet werden kann. Über eine Befestigungsstange 20 mit einer Radialstellklemme 19 sowie einer Befestigungsvorrichtung 21 zur Fixierung der Unterschenkelbeinplatte 22 im erforderlichen Winkel wird dieselbe mit dem nicht gezeigten Operationstisch verbunden. Mit 23 ist eine Aufnahme für die Unterschenkelbeinplatte 22 mit Befestigungseinrichtung für das nicht gezeigte Zugspindelaggregat bezeichnet. Die seitlichen Geräteschienen einschließlich Befestigungen sind mit 24 bezeichnet und dienen der Längsverschiebung des Zielgerätes 90, das in der gewünschten Stellung durch die Radialstellklemme 25 befestigt wird. Mit 26 ist ein Aufnahmeloch zur Befestigung der Teleskopstütze 40 bezeichnet, die in Fig. 5 mit der Teleskopstange 41 und Führungsstange 43, sowie Bodenplatte 44 gezeigt wird. Die Feststellung der geeigneten Höhe wird durch die Arretierungsschraube 42 bewirkt.

In Fig. 4 sind ebenfalls zwei Ansichten der erfindungsgemäß eingesetzten Oberschenkelunterlage 30 in Form einer Beinplatte gezeigt. Mit 31 ist die Aufnahme für diese Oberschenkelbeinplatte 30 zur Befestigung am nicht gezeigten Zugspindelaggregat gezeigt. Diese Aufnahme 31 kann an das jeweilige Operationstischsystem entsprechend angepaßt werden. Denkbar sind hier runde und quadratische Aufnahmeteile 31, die durch die Arretierungsschraube 33 fest verbunden werden. Mit 32 sind seitliche Geräteschienen einschließlich Befestigungseinrichtung bezeichnet.

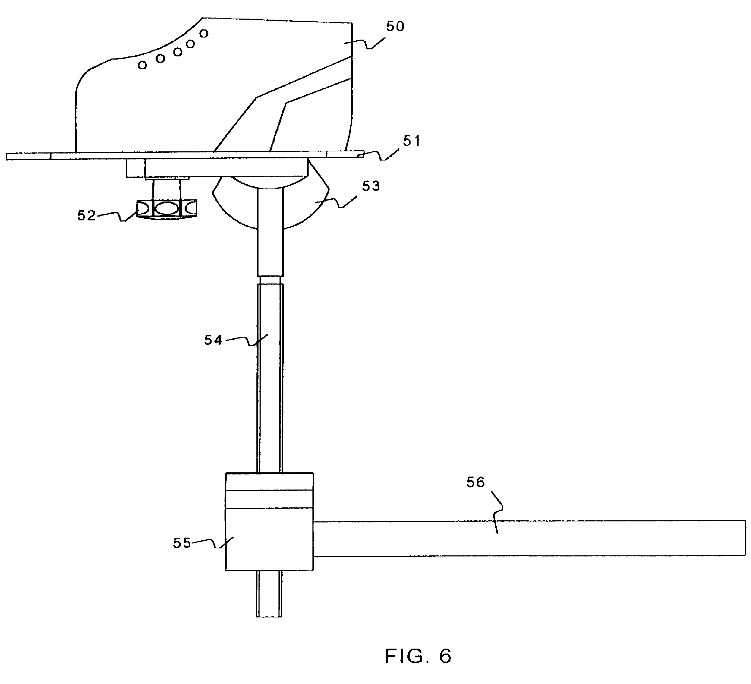

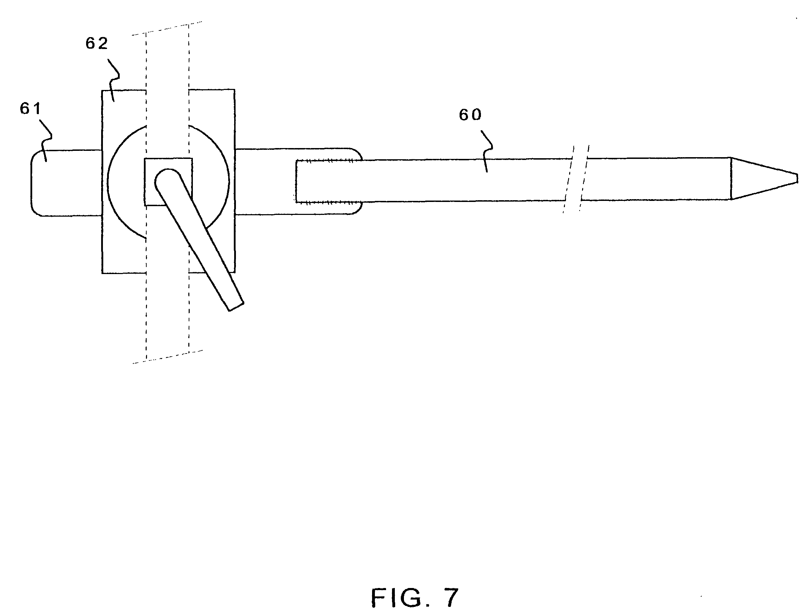

In Fig. 6 ist das Zuggerät bzw. Zugspindelaggregat mit Schuh im Ausschnitt dargestellt. Es besteht aus einem Extensionsschuh 50, der als Schnürschuh mit verkürzter Schaftlänge ausgebildet ist, um die tiefstmögliche distale Verriegelung vornehmen zu können, sowie die Durchführung der anterior-posterior Verriegelung. Er ist an einer Befestigungsplatte 51 mittels einer Arretierungsschraube 52 befestigt. Die Neigung des Extensionsschuhes 50 kann über die Neigungseinstellung 53 zusammen mit der Spindel 54 eingestellt werden, die mit einem Extensionszylinder 55 und einer Befestigungsstange 56 verbunden ist, die in eine der Befestigungsvorrichtung 23 der Tibiaunterlagen 19 bis 26 gesteckt und arretiert wird. In Fig. 7 ist eine Befestigungsstange 60 zum Einsetzen in die autoklavierbare Radialstellklemme 25 gezeigt. Sie weist eine angeschweißte Geräteschiene 61 auf. An dieser vertikalen Stange 60 wird mittels einer autoklavierbaren Radialstellklemme 62 die Befestigungsstange 1 des Zielgerätes 90 derart befestigt, daß sie um 90° horizontal gedreht und somit waagerecht über der Unterschenkelbeinunterlage 22 positioniert ist. Sie befindet sich quasi in 'L-Form' über der Unterlage. In dieser Stellung ist das Zielgerät bei anterior-posterior verlaufenden Verriegelungen verwendbar. Dabei behält sie alle dem System eigenen Freiheitsgrade und ermöglicht die Grobpositionierung durch das Kugelgelenk und Feinpositionierung durch den Steuerkopf. Die Befestigung an der Tibiaunterlage 22 - und nicht am Operationstisch - ist erforderlich, um die zu operierende Extremität aus dem röntgenstrahlenundurchlässigen Bereich des OP-Tisches zu bringen. Außerdem wird an der Tibiaunterlage 22 das Zuggerät 50 bis 56 befestigt und die Extremität (nicht gezeigt) dadurch während des Verriegelungsvorganges fixiert.

In Fig. 8 wird eine Schemaskizze für den vollständigen Gerätaufbau zur distalen Verriegelung am Unterschenkel gezeigt.

Das obere Ende der Tibiaunterlage 22 wird mit einer, an der Befestigungsstange 20 befindlichen Radialstellklemme 19 an einer Geräteschiene des OP-Tisches 65 befestigt. Das untere Ende der Tibiaunterlage 22 wird mittels eines, in der Geräteschiene 24 befindlichen Aufnahmeloches mit dem Aufnahmezapfen des Statives 40 bis 44 verschraubt.

Der Patient wird gelagert. Je nach Beinlänge des Patienten wird die Befestigungsstange 20 der Tibiaunterlage 22 weit oder weniger weit in Richtung Boden geschoben und mit der Radialstellklemme 19 befestigt. Entsprechend wird die Höhe des Statives 40 bis 44 verstellt. Eine Winkelanpassung kann durch die Verstellung und Arretierung der zwischen Tibiaunterlage 22 und Befestigungsstange 20 befindlichen Flügelmutter 21 vorgenommen werden. Das Zugaggregat 50 bis 56 wird mit seiner Befestigungsstange 56 in eine der Befestigungsvorrichtungen 23 der Tibiaunterlage 19 bis 26 gesteckt und arretiert.

Der Fuß des zu operierenden Beines wird in einem Extensionsschuh 50 befestigt. Die Höhe und Stellung des Extensionsschuhs kann mittels der Befestigungsplatte 51 und der Neigungseinstellung 53 angepaßt werden. In dem man den im Extensionsschuh befindlichen Fuß durch das Drehen des - auf der Zugspindel 54 laufenden - Extensionszylinders spannt wird der Knochenbruch reponiert. Dies geschieht unter Durchleuchtungskontrolle. Anschließend wird der Verriegelungsnagel 70 in die Markhöhle des Knochens eingebracht. Der Verriegelungsnagel 70 wird so gedreht, daß seine Verriegelungslöcher 73, 74 sich bei seitlicher Durchleuchtung kreisrund darstellen. Das Bein ist über das Zugaggregat 50 bis 56 und die Tibiaunterlage 19 bis 26 fest mit dem OP-Tisch verbunden.

Das Zielgerät 1 bis 17 mit einer autoklavierbaren Radialstellklemme 25 an einer der Geräteschienen 24 der Tibiaunterlage 19 bis 26 mittels seiner Befestigungsstange 1 fixiert. Die Schablone 14 bis 17 wird auf den Aufnehmerzapfen 13 gesteckt.

Die Schablone 14 bis 17 wird auf den im Bein befindlichen Knochennagel 70 eingestellt. Ziel ist es, die Schablone 14 so zu plazieren, daß die obere Reihe der Bohrungen 15 der Schablone 14 mit den Löchern des Nagels auf einer Ebene liegen.

Die Grobpositionierung erfolgt über Höhenverstellung der mit der autoklavierbaren Radialstellklemme gehaltenen Befestigungsstange 1 und durch die Einstellung der Gelenkkugel 4 und deren Fixierung durch die Knebelschraube 2, 3. Die Feineinstellung wird über die vertikale und horizontale Stellschraube 7 bzw. 11 vorgenommen. Durch vertikale und horizontale Feineinstellung mittels Steuerkopf wird der Steg zweier Bohrlöcher zentral zu dem ausgesuchten Nagelloch eingestellt. Dies geschieht unter Durchleuchtung.

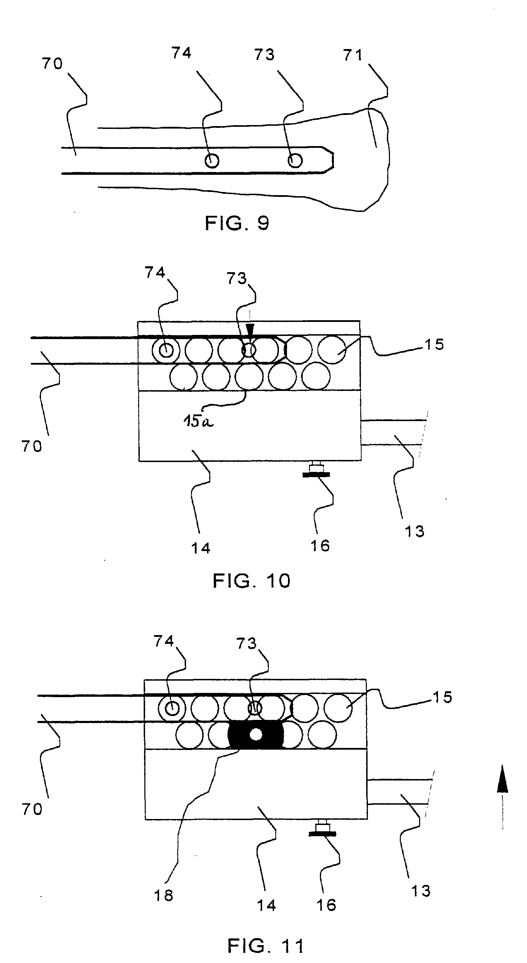

In Fig. 9 ist im Ausschnitt ein Verriegelungsnagel 70 zusammen mit dem distalen Knochenstück 71 gezeigt, der ein erstes und zweites Nagelloch 73, 74 aufweist. Der distale Teil des Nagelsystems wird mittels eines an sich bekannten C-Bogens des Durchleuchtungsgerätes (nicht dargestellt) so eingestellt, daß seine Bohrlöcher sich kreisrund auf dem Bildschirm (nicht dargestellt) darstellen.

Aus Fig. 10 ist ersichtlich, daß der Kopf des Zielgerätes 90 und Durchleuchtung so plaziert wird, daß die obere Reihe der Bohrungen 15 der Bohrschablone 14 mit den Löchern 73, 74 des Nagels 70 auf einer Ebene liegen. Die grobe Positionierung erfolgt mittels der Einstellung über die Gelenkkugel und deren Arretierung durch die Knebelschraube 2 und den Druckknopf 3 zum Arretieren der Gelenkkugel (nicht gezeigt). Die Feineinstellung wird über die vertikale und horizontale Stellschraube 7 bzw. 11 vorgenommen. Durch die vertikale und horizontale Feineinstellung mittels des Steuerkopfes 2 bis 12 wird der Steg zweier Bohrlöcher 15 zentral zum Nagelloch 74 eingestellt. Das darunter liegende Schablonenloch 15a liegt damit genau in der Mitte.

In Fig. 11 ist eine zur Fig. 10 entsprechende Darstellung im Ausschnitt gezeigt, wobei jedoch die zum Nagelsystem passende Reduktionshülse 18 in das untere Loch 15a hineingeschoben wird.

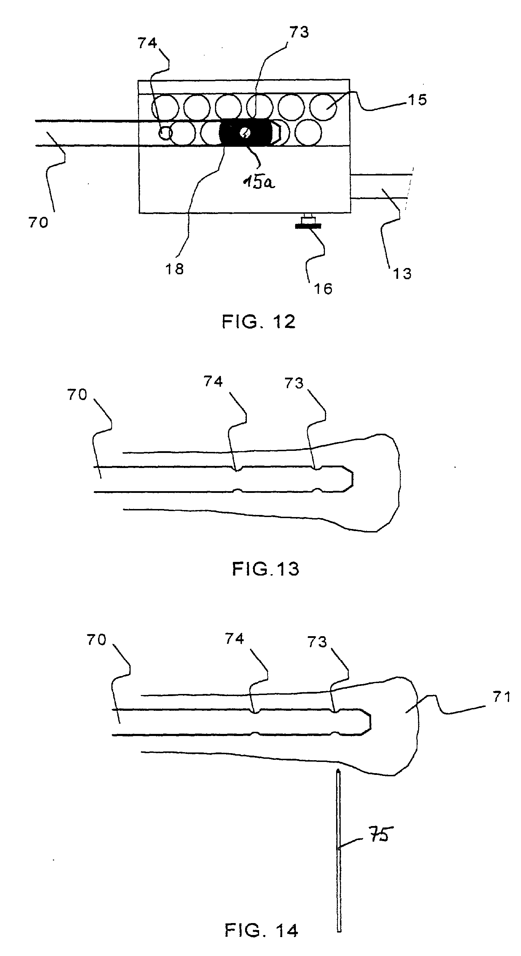

Ebenso zeigt Fig. 12 eine entsprechende Ansicht, in der das reduzierte Bohrloch 15a mit dem Nagelloch 73 deckungsgleich gebracht wird. Durch die horizontale und vertikale Verstellung des Steuerkopfes wird die Einstellung solange optimiert, bis sich das Loch 15a/73 kreisrund darstellt.

In Fig. 13 ist eine der Fig. 9 vergleichbare Ausschnittsansicht von Verriegelungsnagel 70 und distalem Knochenstück 71 gezeigt, diesmal jedoch in der Draufsicht, so daß die Nagellöcher 73 und 74 nur als Einkehrbungen zu sehen sind. Der nicht gezeigte C-Bogen des Durchleuchtungsgerätes steht nun in anterior-posterior Stellung.

Der weitere Verfahrensablauf ist aus den entsprechenden Figuren 14 und 15 zu ersehen, wobei ein Trokar mit 75 bezeichnet ist. Die Reduktionshülse 18 wurde entnommen und mit einem Skalpell (nicht gezeigt) eine Stichinzision durchgeführt. Anschließend wird die Reduktionshülse 18 wieder in das entsprechende Loch der Bohrschablone 14 eingesetzt. Ein Trokar 75 wird in das Loch der Reduktionshülse 18 eingeführt. Unter Durchleuchtung wird der Trokar 75 mittels einer Antriebsmaschine (nicht gezeigt) in das Gewebe eingetrieben. Vor Erreichen des Knochens 71 wird der Vorgang unterbrochen. Die Position der Spitze des Trokars 75 zum Nagelloch 73 wird durch horizontale Verschiebung des Zielgerätes 90 nochmals optimiert, wobei der Eintriebsvorgang solange fortgesetzt wird, bis der Knochen 71 vollständig durchdrungen ist, wie das in Fig. 15 gezeigt wird.

Fig. 16 zeigt dann wiederum einen Ausschnitt mit der Bohrschablone 14, wobei die Antriebsmaschine und die Reduktionshülse 18 entfernt worden sind. Über den Trokar 75 werden eine Arbeitshülse 77 mit Zentrierhülse 76 geschoben, wie dieses durch die schwarze Kreisfläche angedeutet ist. Fig. 17 zeigt diese Situation des Verfahrensablaufes anhand von Knochen 71 und Zentrier- und Arbeitshülse 76 bzw. 77.

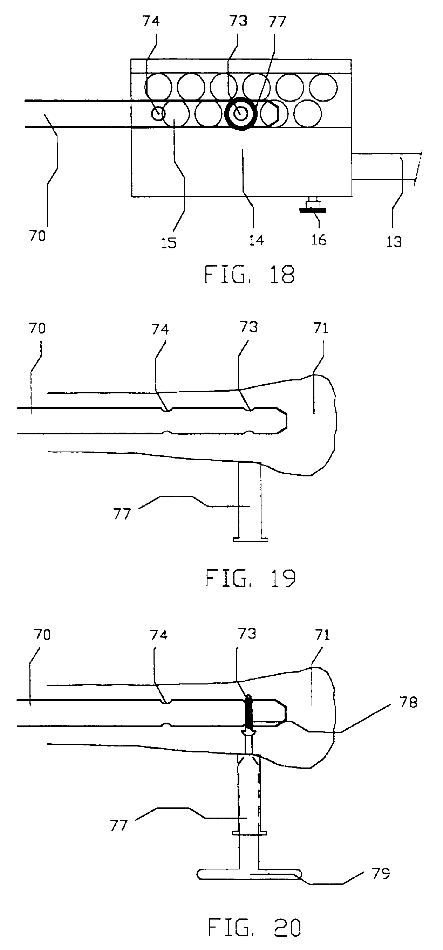

Anschließend werden der Trokar 75 und die Zentrierhülse 76 entfernt. Die Arbeitshülse 77 verbleibt jedoch und hält das um die Bohrung liegende Gewebe frei, wie es in Fig. 18 gezeigt ist. Die gleiche Situation wird in Fig. 19 im Hinblick auf den Knochen 71 und Verriegelungsnagel 70 gezeigt.

Anschließend wird ein nicht gezeigtes Schraubenmeßgerät in die Arbeitshülse 77 eingeführt und die erforderliche Schraubenlänge ermittelt. Fig. 20 zeigt den daran anschließenden Verfahrensschritt, dem eine Schraube 78 der ermittelten Länge durch die Arbeitshülse 77 mittels eines Schraubendrehers 79 unter Durchleuchtung in das Nagelloch 73 des Verriegelungsnagels 70 eingedreht wird.

Abschließend wird die Einstellung des zweiten Bohrloches 74 vorgenommen. Hierzu wird durch horizontale Verschiebung des Zielgerätes 90 die mit dem Nagelloch 74 am besten übereinstimmende Bohrung 15 aufgesucht. Die vertikale Einstellung darf auf keinen Fall verändert werden. Der Bildverstärker bleibt in anterior-posterior Stellung. Auf den erneuten Einsatz der Reduktionshülse 78 wird verzichtet. Der Trokar 75 (für diesen Vorgang nicht dargestellt) mit Arbeits- und Zentrierhülse wird auf das Zentrum des Bohrloches gestellt und mittels Antriebsmaschine eingetrieben. Die vorher beschriebenen Arbeitsschritte werden wiederholt.

## Patentansprüche

1. Distales Zielgerät-System (100) für die Verriegelung von intramedullären Knochennägeln am Femur und/oder Tibia, sowie Humerus und/oder Radius, mit einem Verriegelungsnagelsystem, dadurch gekennzeichnet, daß ein Zuggerät (50-56) mit Spindel (54) und Extensions(hand)schuh (50), sowie röntgenstrahlendurchlässige Unterlagen (22+30) für den Femur und/oder Tibia bzw. den Arm vorgesehen werden, wobei die Femurunterlage (30) direkt und das Zugaggregat (56) und die Tibia- oder Armunterlage (22) über ein Stativ (40-44) am Operationstisch (65) im Gebrauchszustand befestigt sind, und daß ein zur Positionierung in den Strahlengang eines Röntgendurchleuchtungsgerätes bringbares Zielgerät (1-17,90) mit einer auf einem Aufnahmezapfen (13) angeordneten Bohrschablone (14-17), mittels eines Steuerkopfes (2-12) fixiert wird, der Regel- und Arretierungsschrauben (8+12) zur horizontalen und vertikalen Verschiebung der Bohrschablone (14-17) aufweist, am Operationstisch (65) im Gebrauchszustand mittels Befestigungseinrichtungen (1) befestigt ist, wobei mehrere Reduktionshülsen (18) mit unterschiedlichem Durchmesser, sowie Arbeitshülse (77) mit Zentrierhülse (76) und Trokar (75) vorgesehen werden, deren Durchmesser auf die Bohrungen (15) in der Bohrschablone (14-16) zur zielgerichteten Positionierung in Richtung des Nagelloches abgestimmt sind.

2. Distales Zielgerät-System nach Anspruch 1, dadurch gekennzeichnet, daß das Verriegelungsnagelsystem für Knochennägel Teil von etwa 1,5 bis 6 mm Durchmesser geeignet ist.

3. Distales Zielgerät-System nach Anspruch 1 und 2, dadurch gekennzeichnet, daß die Femur- und Tibiaunterlagen (22+30) mit beidseitigen Geräteschienen (24+32) zur Längsverstellung versehen sind, wobei die Femurunterlage (30) eine Halterung (31) mit Arretierung (33) und die Tibiaunterlage (22) eine Befestigungsstange (20) mit Arretierung (21), insbesondere in Form einer Flügelmutter, aufweist.

4. Distales Zielgerät-System nach Anspruch 1 bis 3, dadurch gekennzeichnet, daß die Bohrschablone (14-17) auf dem Aufnahmezapfen hinsichtlich Richtung und Stellung verschieb- und feststellbar angeordnet ist.

5. Distales Zielgerät-System nach Anspruch 1 bis 4, dadurch gekennzeichnet, daß ein Kugelgelenk (2-4) zur groben Positionierung der Bohrschablone (14-17) vorgesehen ist.

6. Distales Zielgerät-System nach Anspruch 1 bis 5, dadurch gekennzeichnet, daß in schwalbenschwanzführung liegende Zahnstangen (5+9) über Rändelschrauben (7+11) zur Feinabstimmung der vertikalen und horizontalen Stellung der Bohrschablone (14-16) vorgesehen sind.

7. Distales Zielgerät-System nach Anspruch 1 bis 6, dadurch gekennzeichnet, daß in an sich bekannter Weise zur Durchführung einer Stichinzision ein Skalpell mit Skalpellhalter vorgesehen ist, und daß unter Einführung einer Schraubenmeßlehre in die Arbeitshülse zur Ermittlung der erforderlichen Schraubenlänge eine Schraubenmeßlehre vorgesehen wird, und daß ein Schraubendreher (79) zum Eindrehen einer Schraube der ermittelten Länge durch die Arbeitshülse vorgesehen wird.

8. Distales Zielgerät-System nach Anspruch 1, dadurch gekennzeichnet, daß eine Antriebsmaschine zum Eintrieb des Trokars oder Bohrers in den Knochen vorgesehen ist.

9. Distales Zielgerät-System nach Anspruch 1 bis 8, dadurch gekennzeichnet, daß der Extensionsschuh als Schnürschuh ohne Spitze mit verkürzter Schaftlänge ausgebildet ist.

10. Distales Zielgerät-System nach Anspruch 1 bis 9, dadurch gekennzeichnet, daß eine vertikale Stange (60-62) an einer Radialstellklemme an den Geräteschienen der Unterschenkelbeinauflage befestigt ist, um den Steuerknopf um 90° in die Horizontale zu verschwenken.
